# EUROPEAN PATENT APPLICATION

(11) **EP 1 935 407 A1**
(43) Date of publication of application: **25.06.2008**
(21) Application number: 08004947.1
(22) Date of filing: 23.04.2003
(51) Int. Cl.: A61K 9/00, A61K 47/32, A61K 47/38, A61K 47/20

(54) **Low viscosity liquid dosage forms**

(30) Priority: 03.12.2002 US 430348 P; 14.04.2003 US 412669
(62) Divisional of application: 03724196.5
(71) Applicant: Elan Pharma International Limited, Athlone Westmeath (IE)
(72) Inventor: Ryde, Tuula A., Malvern PA 19355 (US); Ryde, Niels, Malvern PA 19355 (US); Bosch, William h., Bryn Mawr PA 19010 (US); Pruitt, John D., Collegeville PA 19426 (US); Wertz, Christian F., Landsdale PA 19446 (US)
(74) Representative: Wichmann, Hendrik

(57) **Abstract**

The present invention relates to liquid dosage forms of nanoparticulate active agents having very low viscosities.

## Description

### CROSS-REFERENCE TO RELATED APPLICATIONS

This application claims benefit of U.S. Utility Application No. Unknown, for "Nanoparticulate Formulations of Megestrol," filed on April 14, 2003, and U.S. Provisional Application No. 60/430,348, filed on December 3, 2002.

### FIELD OF THE INVENTION

The present invention relates to low viscosity liquid dosage forms comprising nanoparticulate active agents.

### BACKGROUND OF THE INVENTION

### A. Background Regarding Liquid Dosage Forms

Liquid dosage forms for treatment and diagnosis are useful in a variety of therapies and routes of administration. Liquid dosage forms are particularly useful for patients who cannot swallow or who have difficulty swallowing. Such patients include infant, pediatric, geriatric, some psychiatric patients, and patients requiring enteral feeding.

Because many active agents are poorly water-soluble, developing liquid formulations for oral and parenteral administration for such agents can be problematic. Traditional methods of formulating liquid compositions for oral and parenteral administration include dissolution in non-aqueous solvents and emulsions, loading drugs into liposomes or polymers, use of high or low pH to achieve solubility, and addition of thickening agents (rheology modifiers). Each of these methods, however, presents significant problems.

For example, one method of making particulate suspensions of poorly water-soluble drugs includes using an emulsion having an aqueous phase and a lipophilic phase, such as α-tocopherol and polyethylene glycol (PEG). An alternative means of solubilizing low solubility compounds is direct solubilization in a non-aqueous media, such as an alcohol, dimethylsulfoxide, or triacetin. See e.g. WO 95/11039 which describes using vitamin E and the vitamin E derivative TPGS in combination with ethanol and the immunosuppressant drug cyclosporin. Other representative formulations are provided in U.S. Patent Nos. 5,891,845 and 6,458,373.

Alcohol-containing solutions can be administered with care, but typically result in some degree of vascular irritation and toxicity. Furthermore, pharmaceutical formulations in non-aqueous solvents and solubilizers such as alcohols (ethanol, isopropanol, benzyl alcohol, *etc.*) tend to extract toxic substances, such as plasticizers, from their containers. The current commercial formulation for the anti-cancer drug paclitaxel, for example, consists of a mixture of hydroxylated castor oil and ethanol. This formulation rapidly extracts plasticizers such as di-(2-ethylhexyl)-phthalate from commonly used intravenous infusion tubing and bags. Serious adverse reactions to plasticizers, including respiratory distress, have been reported. Waugh *et al., Am. J. Hosp. Pharmacists, 48*:1520 (1991). Thus the use of such formulations requires special infusion systems which result in extra expense and time.

Conventional liquid formulations of poorly water-soluble active agents are frequently highly viscous, gritty, and require dosages of relatively large volume. This is due, in part, to the relatively large size of the active agent particles and instability of the dispersions used in preparing conventional formulations. In circumstances where the viscosity of water is too low to support poorly water-soluble active agent particles, thickening agents must be added to enhance the stability of the active agent dispersion which prevents aggregation and caking of the active agent particles. Additionally, conventional liquid formulations can be turbid and "gritty" due to the size of the active agent particles upon preparation or due to aggregation and precipitation during storage.

For example, U.S. Patent No. 6,379,692 describes using thickening agents, including hydroxyalkylcelluloses, hyaluronic acid, and polyvinyl pyrrolidone, or mixtures thereof in the preparation of liquid formulations of poorly water-soluble drugs. These compositions have viscosities in the range of 1000 to about 3500 cP. However, liquid active agent formulations with high viscosities are undesirable, as they can be difficult to administer and unpleasant to ingest.

In addition, conventional liquid formulations of megestrol acetate (MEGACE^{®} (Bristol Myers Squibb, Co.) and Megestrol Acetate by PAR Pharmaceuticals, Inc.) have a notably gritty texture and are highly viscous. The micronized megestrol acetate dispersions must be formulated with a flocculating component which aids in resuspension of the megestrol acetate upon settling. Megestrol acetate is a synthetic progestin with progestational effects similar to those of progesterone. It is frequently prescribed as an appetite enhancer for patients in a wasting state, such as HIV wasting, cancer wasting, or anorexia.

Because conventional liquid formulations of poorly water-soluble active agents require the addition of thickening agents and other formulation components, it can be difficult to make these formulations palatable. The requirement of multiple components can result in insufficient masking of disagreeable tastes or odor associated with the active agent. Also, because viscous solutions are retained in the mouth longer, a liquid dosage form having a pleasant taste and smell is important with a highly viscous dosage form.

Liquid dosage forms having low viscosity and small active agent particle size are also desirable for parenteral administration. Viscous solutions can be problematic in parenteral administration because these solutions require a slow syringe push and can stick to tubing. Further, it is generally unsafe to administer intravenous formulations that have a particle size greater than about 2000 nm.

Highly viscous solutions are also difficult to dispense. Viscous solutions can be difficult to pour, especially if the product is refrigerated. Highly viscous solutions cannot be used intravenously.

### B. Conventional Solid Dose Formulations

Formulating solids into tablets can result in large, difficult to swallow tablets. It would be desirable to reformulate such solid dosage forms into a liquid having a low viscosity. Such a reformulated dosage form would be particularly beneficial to patient populations which have difficulty in swallowing tablets, such as infants, pediatrics, and the elderly.

Pediatric patients have difficulty swallowing until they reach the age of about 10-16 years old. Younger pediatric patients generally take either chewable tablets, crush and mix regular tablets with food/juice, or take a liquid dosage form. Chewable tablets, generally a good dosage form, do not always sufficiently mask the taste of the active agent. Crushing and mixing regular tablets with food or juice is time-consuming, messy, and not always practical. A practical and new dosage form would be of value for these patients.

With advancements in medical science and the focus on healthy lifestyles, there is projected growth of the elderly population in the U.S. and abroad. Currently, the U.S. population of persons 65 years of age or older receives nearly 30% of the medications prescribed. Moreover, it is anticipated that there may be a rise in the demand for drugs by the elderly. In spite of the disproportionately large demand for prescription pharmaceuticals among the elderly, relatively little attention has been directed to meeting the unique pharmacotherapeutic needs of this age group.

Many older patients experience difficulty in swallowing tablets or capsules and yet the vast majority of dosage forms administered to the elderly are tablets or capsules. Uncoated tablets are convenient and economical to manufacture but are often difficult to swallow and frequently cause discomfort by "hanging" in the throat. Coated tablets and capsules are somewhat easier to swallow but with increasing age and the large number of drug products that are administered to a single individual, this is a source of apprehension. Conventional liquid dosage forms are relatively easy to administer but often do not taste good, occupy large volumes of space per dosage unit, and possess stability problems. A practical and new dosage form would be of value for these patients as well as others.

### C. Background Regarding Nanoparticulate Active Agent Compositions

One solution to the problem of preparing poorly water-soluble active agents previously suggested is to provide active agent particles in the submicron size range, as described in U.S. Patent No. 5,145,684 ("the '684 patent"), specifically incorporated herein by reference. Such particles can be readily prepared and do not appreciably flocculate or agglomerate due to interparticle attractive forces. The '684 patent does not teach liquid dosage forms having low viscosities.

Methods of making nanoparticulate compositions are described, for example, in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate lododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of lbuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive Nanoparticulate Compositions Having Cationic Surface Stabilizers;" 6,431,478 for "Small Scale Mill;" and 6,432,381 for "Methods for Targeting Drug Delivery to the Upper and/or Lower Gastrointestinal Tract," all of which are specifically incorporated by reference. In addition, U.S. Patent Application No. 20020012675 A1, published on January 31, 2002, for "Controlled Release Nanoparticulate Compositions," describes nanoparticulate compositions, and is specifically incorporated by reference.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter."

There is a need in the art for liquid dosage forms capable of high dose loading, yet having a low viscosity, resulting in a water-like composition. Such a composition may result in better patient compliance as compared to active agents presented in large tablets or large volume and viscous liquid forms. The present invention satisfies these needs.

### SUMMARY OF THE INVENTION

The present invention is directed to low viscosity liquid dosage forms, capable of high dose loading, comprising nanoparticulate active agents. The low viscosity liquid dosage forms of the invention comprise: (1) particles of at least one active agent in combination with (2) at least one surface stabilizer and (3) preferably at least one pharmaceutically acceptable carrier or excipient. The active agent particles have an effective average particle size of less than about 2 microns and the liquid dosage form has a viscosity of less than about 2000 mPa·s at a shear rate of 0.1 (1/s). In other embodiments of the invention the liquid dosage form has a viscosity approaching the viscosity of water, *i.e.,* a viscosity of 1 mPa·s.

Yet another aspect of the invention is directed to a method of improving a conventional solid or liquid dosage form of an active agent. Such a method may comprise identifying a conventional solid dosage form having at least one undesirable trait, such as a large tablet size, followed by reformulating the dosage form into a liquid dosage form according to the invention. Similarly, such a method may comprise identifying a conventional liquid dosage form having at least one undesirable trait, such as high viscosity, followed by reformulating the dosage form into a liquid dosage form according to the invention.

The invention also encompasses a method of making low viscosity liquid dosage forms comprising nanoparticulate active agents. The method comprises contacting particles of at least one active agent with at least one surface stabilizer and, preferably, at least one pharmaceutically acceptable excipient or carrier, for a time and under conditions sufficient to form a liquid dosage form having a viscosity of less than about 2000 mPa·s at a shear rate of 0.1 (1/s). In other embodiments of the invention the liquid dosage form can have a viscosity approaching the viscosity of water, *i.e.,* a viscosity of 1 mPa·s. The active agent particles have an effective average particle size of less than about 2 microns.

A further aspect of the invention is a method of treating a subject in need with a low viscosity liquid dosage form of the invention. The method comprises administering to the subject an effective amount of a low viscosity liquid dosage form according to the invention.

### BRIEF DESCRIPTION OF THE DRAWINGS

FIG. 1 illustrates viscosity in units of mPa·s as a function of concentration of a low viscosity dosage form of the invention. Circles indicate the experimental values and a line illustrates the expected trend.

FIG. 2 shows the viscosity - shear rate curves for two commercial samples of megestrol, 40 mg/mL (Bristol Myers Squibb Co.) (■) and 40 mg/mL (PAR Pharmaceuticals) (◆).

FIG. 3 shows the viscosity - shear rate curves for a commercial sample of naproxen (NAPROSYN^{®}) and two dispersions of nanoparticulate naproxen.

FIG 4. shows the viscosity - shear rate curve for a nanoparticulate dispersion of Compound A, which is a COX-2 inhibitor.

### DETAILED DESCRIPTION OF THE PREFERRED EMBODIMENTS

The present invention relates to liquid dosage forms of nanoparticulate active agents having very low viscosities. Depending upon the active agent, the liquid dosage forms of the invention may be particularly useful as dosage forms for poorly water-soluble active agents in dosage forms (liquid or solid) requiring a high concentration of active agent. Poorly water-soluble active agents tend to be eliminated from the gastrointestinal tract before being absorbed into the circulation.

The liquid dosage forms of the invention comprise one or more nanoparticulate active agents which can be readily prepared and do not appreciably flocculate or agglomerate due to interparticle attractive forces. The active agents preferably are those in need of a low viscosity and/or a high dose loading liquid dosage form.

The liquid dosage forms of the invention provide significant advantages over conventional viscous liquid dosage forms or large solid dosage forms. The low viscosity and silky texture of the liquid dosage forms of nanoparticulate active agents results in advantages in both preparation and use. Depending on the active agent, these advantages may include for example: (1) high dose loading; (2) improved performance characteristics for oral, intravenous, subcutaneous, or intramuscular injection; (3) avoidance of organic solvents or pH extremes; (4) longer active agent dose retention in blood and tumors for some active agents; (5) elimination of fed-fasted effects; (6) more rapid absorption of active agents; (7) better patient compliance due to the perception of a lighter formulation which is easier to consume and digest; (7) ease and accuracy of dispensing due to low viscosity; (8) potential smaller dose volume resulting from a higher concentration of active agent ingredient, and thus less volume for the patient to consume; (9) easier overall formulation concerns; (10) liquid dosage forms suitable for parenteral administration; (11) the liquid dosage forms can be sterile filtered; and (12) increased bioavailability of an active agent.

In the present invention, the liquid dosage forms have a low viscosity and, preferably, the viscosity demonstrates Newtonian behavior. Typically, the nanoparticulate active agents are produced in a size range where it is believed Brownian motion keeps the particles suspended, obviating the use of thickening agents and additives to prevent settling or caking. Thus, an especially preferred embodiment is one in which no thickening or flocculating agents are required to render the liquid dosage form stable.

Another important aspect of the invention is that the liquid dosage form is "water-like" and "silky." As such, a preferred embodiment of the invention comprises a liquid dosage form that is substantially less gritty than a conventional liquid dosage form of the same active agent. "Gritty," as used herein refers to the property of particulate matter that can be seen with the naked eye or that which can be felt as "gritty." The liquid dosage forms of the invention can be poured out of or extracted from a container as easily as water, whereas a conventional (*i.e.,* non-nanoparticulate or solubilized active agent) liquid dosage form of the same active agent loading is notably more "sluggish".

It is desirable to have a liquid dosage form for oral administration that is palatable, silky in texture, and which has a low viscosity at high dose loading levels. Such water-like formulations can result in increased patient compliance because the formulation is more agreeable to consume as compared to a large solid dose form ("horse pill") or highly viscous liquid dosage form. These properties are especially important when considering juvenile patients, terminally ill patients, and patients suffering from gastrointestinal tract dysfunction or other conditions where nausea and vomiting are symptoms. For example, patients suffering from cancer or AIDS-related complications are commonly hypermetabolic and, at various stages of disease, exhibit gastrointestinal dysfunction. Additionally, drugs used to treat these conditions often cause nausea and vomiting. Viscous or gritty formulations, and those that require a relatively large dosage volume, are not well tolerated by patient populations suffering from wasting associated with these diseases because the formulations can exacerbate nausea and encourage vomiting.

The liquid dosage forms of the invention comprise stable nanoparticulate active agents. A typical useful concentration for an active agent such as megestrol acetate is between about 50 mg/mL and about 250 mg/mL. This range will vary with the active agent used and the dosage required. The maximal dose loading of the dosage forms of the invention is significantly higher than the maximal dose loading provided by conventional prepared formulations of the same active agents. A dose loading which is double or more than that utilized in conventional liquid dosage forms of the same active agent are expected to be useful.

The liquid dosage forms of the invention can be formulated for dosages in any volume, but are preferably formulated into equivalent or smaller volumes than existing conventional liquid dosage forms of the same active agent (*i.e.,* non-nanoparticulate or solubilized active agent formulations). For example, the invention encompasses liquid dosage forms formulated into a volume which is at least half that of an existing conventional liquid dosage form of the same active agent. Even smaller dosage volumes are also possible.

Liquid dosage forms having low viscosity and small active agent particle size are desirable for parenteral administration. Viscous solutions can be problematic in parenteral administration because such solutions require a slow syringe push and can stick to tubing. Further, it is unsafe to administer intravenous formulations that have a particle size greater than about 2000 nm. Moreover, conventional formulations of poorly water-soluble active agents tend to be unsafe for intravenous administration techniques, which are used primarily in conjunction with highly water-soluble substances.

Highly viscous and turbid solutions are also difficult to accurately dispense. Viscous solutions can be difficult to pour, especially if the product is refrigerated.

Low viscosity liquid dosage forms of nanoparticulate active agents can be sterile filtered, obviating the need for heat sterilization, which can harm or degrade many active agents as well as result in crystal growth and particle aggregation. Sterile filtration can be difficult because of the required small particle size of the composition. Filtration is an effective method for sterilizing homogeneous solutions when the membrane filter pore size is less than or equal to about 0.2 microns (200 nm) because a 0.2 micron filter is sufficient to remove essentially all bacteria. Sterile filtration is normally not used to sterilize conventional suspensions of micron-sized active agents because the active agent particles are too large to pass through the membrane pores. A sterile liquid dosage form is particularly useful in treating immunocompromised patients, infants or juvenile patients, and the elderly, as these patient groups are the most susceptible to infection caused by a non-sterile liquid dosage form.

An additional advantage provided by the liquid dosage forms of the invention may be enhanced bioavailability, which is particularly important in treating conditions involving improper gastrointestinal tract function, such as gastric stasis. Bioavailability is the degree to which an active agent becomes available to the target tissue after administration. Many factors can affect bioavailability, including the dosage form and dissolution rate of the active agent. Poor bioavailability is a significant problem encountered in the development of pharmaceutical compositions, particularly those comprising a poorly water-soluble active agent. Poorly water-soluble active agents tend to be eliminated from the gastrointestinal tract before being absorbed into the circulation of the patient.

For active agents having a dissolution-rate limited bioavailability, a faster rate of dissolution is associated with greater bioavailability and a slower rate of dissolution is associated with a lower bioavailability. In such cases, bioavailability is related to the surface area of an administered active agent and, therefore, bioavailability increases with a reduction in the particle size of the dispersed active agent. Increasing the rate of dissolution of an active agent is often desirable because it can result in an increased rate of absorption *in vivo,* increased bioavailability, and decreased variability in absorption of the active agent.

Unless indicated otherwise, all technical and scientific terms are used in a manner that conforms to common technical usage. Standard techniques are used for analytical chemistry, organic synthetic chemistry, chemical syntheses, chemical analysis, and pharmaceutical formulation and delivery. Absent an indication to the contrary, the techniques and procedures in question are performed according to conventional methodology.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein, "stable" will be understood by persons of ordinary skill in the art and will vary to some extent depending on the context in which it is used. If there are uses of the term which are not clear to persons of ordinary skill in the art given the context in which it is used, "stable" will mean that the particles do not appreciably flocculate or agglomerate due to interparticle attractive forces or otherwise increase in particle size.

As used herein, "standard liquid dosage form", "conventional liquid dosage form," or variations thereof refer to liquid dosage forms of either solubilized or microparticulate active agents.

### A. Compositions

The liquid dosage forms of the invention comprise stable dispersions of nanoparticulate active agents. A stable dispersion allows high solids loading while maintaining homogeneity and consistent rheological behavior. For dispersions, stability implies that the particles of the invention are dominated by Brownian motion, whereas inertia and gravity have a larger effect on the behavior of suspensions of larger particles, such as those in conventional liquid dosage forms of the same active agents.

The liquid dosage forms of the invention comprise at least one nanoparticulate active agent and at least one surface stabilizer associated with the surface of the active agent. Surface stabilizers useful herein are associated with the surface of the nanoparticulate active agent, but do not chemically react with the active agent or itself. Individually adsorbed molecules of the surface stabilizer are essentially free of intermolecular crosslinkages.

The liquid dosage forms of the invention can also include one or more non-toxic physiologically acceptable excipients, carriers, adjuvants, or vehicles, collectively referred to as "excipients." The compositions can be formulated for parenteral injection (*e.g.*, intravenous, intramuscular, or subcutaneous), oral administration, vaginal, nasal, rectal, ocular, local (ointments or drops), buccal, intracisternal, intraperitoneal, or topical administration, and the like. Preferably, the liquid dosage forms of the invention are formulated for oral or injectable administration.

### 1. Viscosity of the Liquid Dosage Forms

The invention provides liquid dosage forms having a low viscosity. Viscosity is concentration and temperature dependent. Typically, a higher concentration results in a higher viscosity, while a higher temperature results in a lower viscosity. Viscosity as defined herein refers to a measurements taken at about 20°C. (The viscosity of water at 20°C is 1 mPa·s.) The invention encompasses equivalent viscosities measured at different temperatures.

Low viscosity is an important feature for oral administration of liquids when treating patients with difficulty in swallowing or in patients who suffer from symptoms including nausea and vomiting. Low viscosity is an especially important feature in injectable formulations. Typically the viscosity of the liquid dosage forms of the invention, at a shear rate of 0.1 (1/s), are from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to about 1 mPa·s.

The viscosity of the liquid dosage forms of the invention is preferably less than the viscosity of a standard or conventional liquid dosage form of the same active agent, at about the same concentration of active agent. Preferably the viscosity of the liquid dosage forms of the invention is less than about 1/200, less than about 1/100, less than about 1/50, less than about 1/25, or less than about 1 /10 of the viscosity of a conventional liquid dosage forms of the same active agent, at about the same concentration per ml of the active agent.

In other embodiments of the invention, preferably the viscosity of the liquid dosage forms of the invention is less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, or less than about 90% of the viscosity of a standard conventional liquid dosage form of the same active agent at about the same concentration per ml of active agent.

The invention also provides low viscosity liquid dosage forms of nanoparticulate active agents that do not require thickening agents..

### 2. Drug Particles

The low viscosity liquid dosage forms of the invention include at least one active agent, also referred to as a "drug", "active ingredient", "therapeutic agent," or "diagnostic agent." An active agent can be a pharmaceutical or a diagnostic agent such as a contrast agent or any other type of diagnostic material. The therapeutic or diagnostic agent exists as a discrete, crystalline phase, a semi-crystalline phase, an amorphous phase, a semi-amorphous phase, or a mixture thereof.

The invention can be practiced with a wide variety of active agents. The active agent is preferably present in an essentially pure form and is poorly soluble and dispersible in at least one liquid media. "Poorly soluble active agents" or "poorly soluble drugs" as used herein means those having a solubility in a liquid dispersion media of less than about 30 mg/ml under ambient conditions. In other embodiments of the invention, the active agent has a solubility in the liquid dispersion media of less than about 10 mg/ml or less than about 1 mg/ml. A preferred liquid dispersion media is water. However, the invention can be practiced with other liquid media in which an active agent is poorly soluble and dispersible including, for example, aqueous salt solutions, safflower oil, and solvents such as ethanol, t-butanol, hexane, and glycol. The pH of aqueous dispersion media can be adjusted by techniques known in the art.

The active agent preferably is of the kind that results in a tablet size that is too large to be optimal. A preferred active agent can also be one in which the viscosity of water is too low to support the poorly water-soluble active agent particles, thus necessitating the addition of thickening agents to conventional liquid dosage forms to enhance the stability and prevent aggregation and caking of the active agent particles.

The active agent can be selected from a variety of known classes of drugs, including, for example, COX-2 inhibitors, anticancer agents, NSAIDS, proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.

Illustrative nutraceuticals include, but are not limited to, dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.

A description of these classes of active agents and a listing of species within each class can be found in Martindale, *The Extra Pharmacopoeia,* 31^{st} Edition (The Pharmaceutical Press, London, 1996), specifically incorporated herein by reference. The drugs can be commercially available and/or can be prepared by techniques known in the art.

### 3. Surface Stabilizers

Useful surface stabilizers which can be employed in the invention include, but are not limited to, known organic and inorganic pharmaceutical excipients. Such compounds include various polymers, low molecular weight oligomers, natural products, and surfactants. Surface stabilizers include nonionic, anionic, cationic, and zwitterionic surfactants.

Representative examples of surface stabilizers include hydroxypropyl methylcellulose, hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g.*, macrogol ethers such as cetomacrogol 1000), polyoxyethylene castor oil derivatives, polyoxyethylene sorbitan fatty acid esters (*e.g.*, the commercially available Tweens^{®} such as e.g., Tween 20^{®} and Tween 80^{®} (ICI Speciality Chemicals)); polyethylene glycols (*e.g.*, Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hydroxypropylmethylcellulose phthalate, noncrystalline cellulose, magnesium aluminum silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g.*, Pluronics F68^{®} and F108^{®}, which are block copolymers of ethylene oxide and propylene oxide); poloxamines (*e.g.*, Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-IOG^{®} or Surfactant 10-G^{®} (Olin Chemicals, Stamford, CT); Crodestas SL-40^{®} (Croda, Inc.); and SA90HCO, which is C₁₈H₃₇CH₂(CON(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅-dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™, tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quaternized polyoxyethylalkylamines, MIRAPOL^{™} and ALKAQUAT^{™} (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Particularly preferred nonpolymeric primary stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n > 1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCI, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference. The surface stabilizers are commercially available and/or can be prepared by techniques known in the art.

### 4. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quaternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose such as Avicel^{®} PH101 and Avicel^{®} PH 102, lactose such as lactose monohydrate, lactose anhydrous, and Pharmatose^{®} DCL21, dibasic calcium phosphate such as Emcompress^{®}, mannitol, starch, sorbitol, sucrose, and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

An important aspect of the invention is that the liquid dosage form has a low viscosity. A preferred embodiment of the invention is a composition that does not contain thickening agents or other excipients that result in significant thickening of the liquid composition.

### 5. Nanoparticulate Active Agent Particle Size

The liquid dosage forms of the invention comprise nanoparticulate active agents having an effective average particle size of less than about 2 microns (*i.e.,* 2000 nm). In other embodiments of the invention, the active agent can have an effective average particle size of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

For injectable liquid dosage forms, the one or more active agents preferably have an effective average particle size of no greater than 250 nm.

By "an effective average particle size of less than about 2 microns" (or 1900 nm, 1800 nm, *etc*.) it is meant that at least 50% of the particles by weight have a particle size of less than about 2 microns (or 1900 nm, 1800 nm, *etc.*) when measured by the above-noted techniques. In other embodiments of the invention, at least about 70%, about 90%, or about 95% of the active agent particles have a particle size less than the effective average, *e.g*., less than about 2 microns, less than about 1900 nm, less than about 1800 nm, less than about 1700 nm, *etc.*

### 6. Concentration of Nanoparticulate Active Agent and Surface Stabilizer

The relative amounts of one or more active agents and one or more surface stabilizers can vary widely. The optimal amount of the individual components can depend, for example, upon the particular active agent selected, the hydrophilic lipophilic balance (HLB), melting point, and surface tension of water solutions of the surface stabilizer, the ability of a given compound to increase the viscosity of the composition, *etc*.

The concentration of the one or more active agents can vary from about 99.5% to about 0.001 %, from about 95% to about 0.1 %, or from about 90% to about 0.5%, by weight, based on the total combined dry weight of the one or more active agents and at least one surface stabilizer, not including other excipients.

The concentration of the at least one surface stabilizer can vary from about 0.5% to about 99.999%, from about 5.0% to about 99.9%, or from about 10% to about 99.5%, by weight, based on the total combined dry weight of the one or more active agents and at least one surface stabilizer, not including other excipients.

### B. Methods of Improving Conventional Solid or Liquid Dosage Forms

The invention also encompasses methods of improving a conventional solid or liquid dosage form of an active agent.

Conventional solid dosage forms may have one or more undesirable traits, such as poor dose uniformity, low dose loading, large size ("horse pills"), poor bioavailability, slow onset of activity; poor active agent retention in blood and tumors, significant fed-fasted variability, *etc*.

Similarly, conventional liquid dosage forms may have one or more undesirable traits, such as high viscosity, poor taste, grittiness, poor bioavailability, slow onset of activity, presence of thickening agents, poor dose loading, poor performance characteristics for oral, intravenous, subcutaneous, or intramuscular injection, presence of organic solvents, presence of a pH extreme, poor active agent retention in blood and tumors, significant fed-fasted variability, high dose volume, poor suitability for parenteral administration, an inability to be sterile filtered, *etc.* Such a conventional liquid dosage form may also be one in which the viscosity of water is too low to support the poorly water-soluble active agent particles, thus necessitating the addition of thickening agents to the conventional liquid dosage form to enhance the stability and prevent aggregation and caking of the active agent particles.

A method of improving a conventional solid or liquid oral dosage form of an active agent comprises identifying a conventional solid or liquid dosage form having at least one undesirable trait, followed by reformulating the conventional dosage form into a liquid dosage form according to the invention.

### C. Methods of Making Low Viscosity Liquid Dosage Forms of Nanoparticulate Active Agent Compositions

The one or more nanoparticulate active agents present in the liquid dosage forms of the invention can be made using, for example, milling, homogenization, or precipitation techniques. Exemplary methods of making nanoparticulate active agent compositions are described in the '684 patent. Methods of making nanoparticulate active agent compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation," all of which are specifically incorporated by reference.

In some embodiments, the nanoparticulate active agent dispersion obtained directly after size reduction of the active agent is too concentrated and difficult to measure to provide a consistent dosage unit. In such cases the nanoparticulate active agent dispersion is typically diluted. The final volume of the dosage unit depends upon the dose of active agent needed such that the volume to be given to the patient is measurable and useful. Preferably, the volume of the dosage of the nanoparticulate composition is less than the volume of the dosage of a conventional formulation of equivalent therapeutic efficacy.

Each of the resultant liquid dosage forms (made using any of the methods described below) can be utilized in any suitable method of administration, such as oral, pulmonary, rectal, ophthalmic, colonic, parenteral, intracisternal, intravenous, intravaginal, intraperitoneal, local, buccal, nasal, and topical administration.

### 1. Milling to Obtain Low Viscosity Liquid Dosage Forms of Nanoparticulate Active Agents

Milling an active agent to obtain a dispersion of nanoparticulate active agent particles comprises dispersing particles of the active agent in a liquid dispersion media in which the active agent is poorly soluble, followed by applying mechanical means in the presence of grinding media to reduce the particle size of the active agent to the desired effective average particle size. The dispersion media can be, for example, water, safflower oil, ethanol, t-butanol, glycerin, polyethylene glycol (PEG), hexane, or glycol. A preferred dispersion media is water.

The active agent particles can be reduced in size in the presence of at least one surface stabilizer. Alternatively, the active agent particles can be contacted with one or more surface stabilizers after attrition. Other compounds, such as a diluent, can be added to the active agent/surface stabilizer composition during the size reduction process. Dispersions can be manufactured continuously or in a batch mode.

Following size reduction of the active agent, any desired excipients can be added to the nanoparticulate active agent dispersion to obtain a liquid dosage form having a viscosity of less than about 2000 mPa·s at a shear rate of 0.1 (1/s). The pharmaceutically acceptable excipient can be the dispersion media in which the active agent particles are milled.

### 2. Precipitation to Obtain Low Viscosity Liquid Dosage Forms of Nanoparticulate Active Agents

Another method of preparing the one or more nanoparticulate active agents present in the liquid dosage forms of the invention is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving an active agent in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means. The resultant nanoparticulate dispersion can be used in all manner of liquid dosage formulations.

Following size reduction of the active agent, any desired excipients can be added to the nanoparticulate active agent dispersion to obtain a liquid dosage form having a viscosity of less than about 2000 mPa·s at a shear rate of 0.1 (1/s).

### 3. Homogenization to Obtain Low Viscosity Liquid Dosage Forms of Nanoparticulate Active Agents

Exemplary homogenization methods of preparing nanoparticulate active agent compositions are described in U.S. Patent No. 5,510,118, for "Process of Preparing Therapeutic Compositions Containing Nanoparticles." Such a method comprises dispersing particles of an active agent in a liquid dispersion media in which the active agent is poorly soluble, followed by subjecting the dispersion to homogenization to reduce the particle size of the active agent to the desired effective average particle size. The active agent particles can be reduced in size in the presence of at least one surface stabilizer. Alternatively, the active agent particles can be contacted with one or more surface stabilizers either before or after attrition. Other compounds, such as a diluent, can be added to the active agent/surface stabilizer composition either before, during, or after the size reduction process. Dispersions can be manufactured continuously or in a batch mode. The resultant nanoparticulate dispersion can be used in all manner of liquid dosage formulations.

Following size reduction of the active agent, any desired excipients can be added to the nanoparticulate active agent dispersion to obtain a liquid dosage form having a viscosity of less than about 2000 mPa·s at a shear rate of 0.1 (1/s). The pharmaceutically acceptable excipient can be the dispersion media in which the active agent particles are milled.

### D. Methods of Using the Low Viscosity Liquid Dosage Forms of the Invention

The liquid dosage forms of the invention can be administered to a subject via any conventional method including, but not limited to, orally, rectally, ocularly, parenterally (*e.g.*, intravenous, intramuscular, or subcutaneous), intracisternally, pulmonary, intravaginally, intraperitoneally, locally (*e.g.*, ointments or drops), or as a buccal or nasal spray. As used herein, the term "subject" is used to mean an animal, preferably a mammal, including a human or non-human. The terms "patient" and "subject" may be used interchangeably.

The smooth, low viscosity, liquid dosage forms of the invention are especially useful in oral administration of active agents to treat subjects suffering from symptoms such as nausea and vomiting. As such, in one embodiment of the invention the liquid dosage forms are used to treat, for example, motion sickness, emesis related to cytotoxic drugs, gastritis, ulcers, dyspepsia, gastroenteritis, including collitis and food poisoning, inflammatory bowel disease, Crohn's disease, migraine headaches, pain and/or inflammation, and any other condition which is accompanied by the symptoms of nausea and vomiting.

**[0094]** For example, COX-2 inhibitors and NSAIDS are known to mediate inflammation and pain present in arthritis and pain. The two most common forms of the arthritis, osteoarthritis and rheumatoid arthritis, have the greatest public health implications, according to the Arthritis Foundation. Other common forms arthritis and related conditions include juvenile arthritis, gout, ankylosing spondylitis, systemic lupus erythematosus, bursitis, tendinitis and myofascial pain, carpal tunnel syndrome, fibromyalgia syndrome, infectious arthritis, psoriatic arthritis, reiter's syndrome, and scleroderma. In addition, the COX-2 enzyme is believed to play an important role in cancer, kidney disease, osteoporosis, Alzheimer's disease, and familial adenomatous polyposis. Smooth, low viscosity, liquid dosage forms of nanoparticulate COX-2 inhibitor and NSAID compositions, according to the invention, can be used to treat such conditions.

Another preferred embodiment of the invention encompasses liquid dosage forms of nanoparticulate active agents in which conventional formulations of the microparticulate or solubilized active agents typically have an unpleasant taste, such as cough suppressants and liquid cold and flu preparations.

The effective amounts of the nanoparticulate active agent of the liquid dosage forms of the invention can be determined empirically and can be employed in pure form or, where such forms exist, in pharmaceutically acceptable salt, ester, or prodrug form. Actual dosage levels of the nanoparticulate active agent in the liquid dosage forms of the invention may be varied to obtain an amount of the active agent that is effective to obtain a desired therapeutic response for a particular composition and method of administration and the condition to be treated. The selected dosage level therefore depends upon the desired therapeutic effect, the route of administration, the potency of the administered active agent, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific ingredient or composition employed; the specific ingredients or composition employed; the age, body weight, general health, sex, and diet of the patient; the time of administration, route of administration, and rate of excretion of the agent; the duration of the treatment; drugs used in combination or coincidental with the specific active agent; and like factors well known in the medical arts.

"Therapeutically effective amount" as used herein with respect to an active agent dosage unit, shall mean that amount of active agent that provides the specific pharmacological response, *i.e.,* therapeutic or prophylactic, for which the active agent is administered in a significant number of subjects in need of such treatment. It is emphasized that "therapeutically effective amount," administered to a particular subject in a particular instance will not always be effective in preventing or treating the diseases described herein, even though such dosage is deemed a "therapeutically effective amount" by those skilled in the art. It is to be further understood that active agent dosages are, in particular instances, measured as oral dosages, or with reference to active agent levels as measured in blood.

Compositions suitable for parenteral injection may include physiologically acceptable sterile aqueous or nonaqueous solutions, dispersions, suspensions or emulsions, and sterile powders for reconstitution into sterile injectable solutions or dispersions. Proper fluidity can be maintained, for example, by the use of a coating such as lecithin, by the maintenance of the required particle size in the case of dispersions, and by the use of surfactants.

The liquid dosage forms may also comprise adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Liquid dosage forms for oral administration include pharmaceutically acceptable emulsions, solutions, suspensions, syrups, and elixirs. In addition to the active agent and surface stabilizer, the liquid dosage forms may include inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propylene glycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like. Besides such inert diluents, the liquid dosage form can also include adjuvants, such as wetting agents, emulsifying and suspending agents, sweetening, flavoring, and perfuming agents. In a preferred embodiment, no thickening agent is employed. In another preferred embodiment the liquid dosage forms comprise a minimum of components to enable effective taste masking.

The following example is given to illustrate the present invention. It should be understood, however, that the invention is not to be limited to the specific conditions or details described in this example. Throughout the specification, any and all references to a publicly available document, including a U.S. patent, are specifically incorporated by reference.

The formulations in the example that follows were also investigated using a light microscope. Here, "stable" nanoparticulate dispersions (uniform Brownian motion) were readily distinguishable from "aggregated" dispersions (relatively large, nonuniform particles without motion). Stable, as known in the art and used herein, means the particles don't substantially aggregate or ripen (increase in fundamental particle size).

### Example 1

The purpose of this example was to demonstrate the improved viscosity characteristics of the liquid dosage forms of the invention as compared to conventional liquid dosage forms of the same active agent, megestrol acetate.

Megestrol Acetate is currently marketed by Bristol Myers Squibb, Co. (Megace^{®}) and Par Pharmaceuticals, Inc. The formulations are relatively large volume. For example, both BMS's Megace^{®} and Par Pharmaceuticals' megestrol acetate oral suspension contains 40 mg of micronized megestrol acetate per ml and the package insert recommends an initial adult dosage of megestrol acetate oral suspension of 800 mg/day (20 mL/day). The commercial formulations of megestrol acetate are highly viscous suspensions, which have a relatively long residence time in the mouth and any tubing. Highly viscous substances are not well accepted by patient populations, particularly patients suffering wasting and those that are intubated.

Three liquid dosage forms of nanoparticulate megestrol acetate particles were prepared. The three liquid dosage forms comprised the same relative composition of nanoparticulate megestrol acetate but at different concentrations: 30, 50, and 90 mg/mL of megestrol acetate.

The three liquid dosage forms were prepared by first milling megestrol acetate under high energy milling conditions using a 2 L recirculation mill (Type: LMZ 2; manufactured by Netzsch, Inc. (Exton, PA)) in the presence of a preservative / buffer system consisting of sodium benzoate, citric acid monohydrate, and sodium citrate dihydrate. After milling, the resulting dispersion was diluted with water, sucrose, flavoring, and additional preservative / buffer to prepare dispersions containing 3% (w/w), 5% (w/w), or 9% (w/w) megestrol acetate. The resulting formulations are shown in the table below. The physical stability of the formulations was then monitored at 25°C, 40°C, and 50°C.

| **TABLE 1** | | | | |
|---|---|---|---|---|
| **Summary of the Three Liquid Dosage forms of Nanoparticulate Megestrol Acetate** | | | | |
| | **Concentrated Nanoparticulate Dispersion of Megestrol Acetate** | **Diluted, Flavored Dispersions** | | |
| | | **3% Dispersion** | **5% Dispersion** | **9% Dispersion** |
| API and Excipients | g/kg | g/kg | g/kg | g/kg |
| Megestrol Acetate, USP | 325.000 | 30.000 | 50.000 | 90.000 |
| Hydroxypropyl Methylcellulose, USP | 65.000 | 6.000 | 10.000 | 18.000 |
| Docusate Sodium, USP | 3.250 | 0.300 | 0.500 | 0.900 |
| Sodium Benzoate, USP | 1.214 | 1.826 | 1.777 | 1.681 |
| Sodium Citrate Dihydrate, USP | 0.910 | 0.091 | 0.089 | 0.084 |
| Citric Acid Monohydrate, USP | 0.061 | 1.369 | 1.333 | 1.260 |
| Sucrose, USP | | 50.000 | 50.000 | 50.000 |
| Natural and Artificial Lemon Flavor | | 0.400 | 0.400 | 0.400 |
| Artificial Lime Flavor | | 0.400 | 0.400 | 0.400 |
| Purified Water, USP | 604.600 | 909.614 | 885.500 | 837.280 |

Particle size measurements were used to assess the physical stability. The results of the measurements showed almost no increase in the mean particle size at either 25°C or 40°C, and only a slight increase in the mean particle size at 50°C. 126 days of stability measurements were obtained for the 5% and 9% dispersions and 33 days of stability were obtained for the 3% dispersion, which was prepared at a later date. The results of the stability testing are shown below.

| **TABLE 2** | | | | | | | | | |
|---|---|---|---|---|---|---|---|---|---|
| **Mean Particle Size (nm) of the Three Low Viscosity Liquid Dosage Forms of Nanoparticulate Megestrol Acetate Over Time** | | | | | | | | | |
| | 3% Dispersion | | | 5% Dispersion | | | 9% Dispersion | | |
| | 25°C | 40°C | 50°C | 25°C | 40°C | 50°C | 25°C | 40°C | 50°C |
| | | | | | | | | | |
| 0 days | 148 | 148 | 148 | 169 | 169 | 169 | 169 | 169 | 169 |
| 30 days | | | | 172 | 171 | 187 | 172 | 170 | 179 |
| 33 days | 141 | 144 | 173 | | | | | | |
| 126 days | | | | 171 | 174 | 188 | 168 | 175 | 182 |

The viscosity of the three liquid dosage forms of nanoparticulate megestrol acetate and two commercial formulations of megestrol acetate (Megace^{®} by Bristol Myers Squibb, Co., and megestrol acetate marketed by Par Pharmaceuticals, Inc.) were determined using a rheometer (model CVO-50, Bohlin Instruments). The measurements were performed at a temperature of 20 °C using a double gap (40/50) geometry.

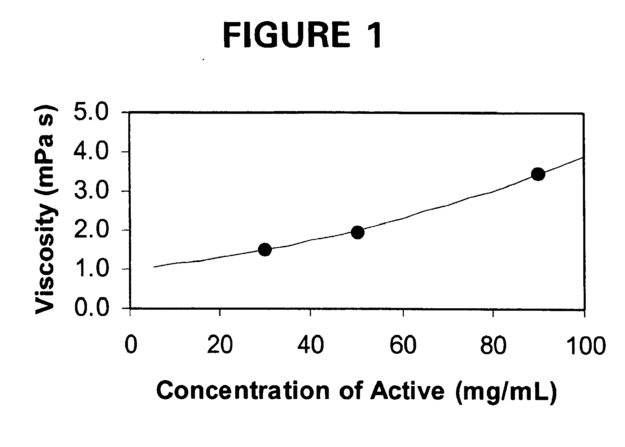

The viscosities of the three liquid dosage forms of nanoparticulate megestrol acetate were found to be nearly Newtonian (*i.e.* the viscosity being independent of shear rate) and were 1.5, 2.0, and 3.5 mPa s for the 30, 50, and 90 mg/mL concentrations, respectively. See Table 3, below, and FIG. 1. The dependence of viscosity on concentration is also shown in FIG. 1.

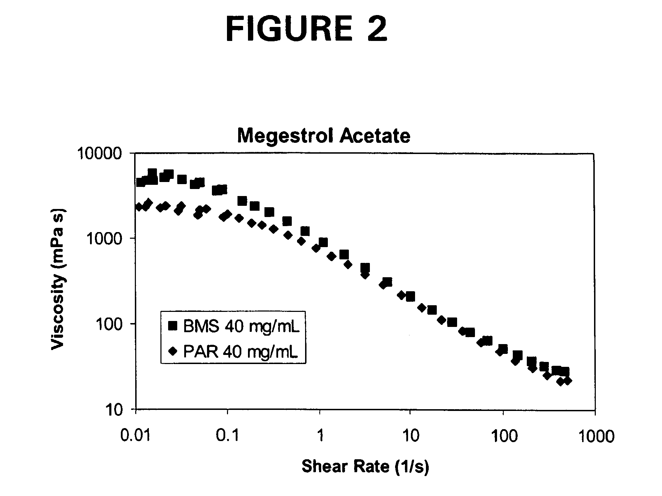

The commercial samples were shear thinning in nature. Such samples cannot be characterized by a single viscosity but rather a series of viscosities measured at different shear rates. This is most conveniently illustrated as viscosity - shear rate curves. FIG. 2 shows the viscosity - shear rate curves for two commercial samples, BMS and PAR, both at an active concentration of 40 mg/mL.

The viscosities of the two commercial samples of megestrol acetate, BMS and PAR, and the three liquid dosage forms of nanoparticulate megestrol acetate are compared in Table 3. Viscosities are in units of mPa·s. Sheer rates of 1-100 approximate those encountered in chewing and swallowing.

**TABLE 3**

| **Shear Rate** | **Commercial Samples** | | **Low Viscosity Liquid Dosage Forms*** | | |
|---|---|---|---|---|---|
| **s⁻¹** | **BMS (40 mg/ml) (mPa·s)** | **PAR (40mg/ml) (mPa·s)** | **30 mg/mL (mPa·s)** | **50 mg/mL (mPa·s)** | **90 mg/mL (mPa·s)** |
| 0.1 | 4010 | 2860 | 1.5 | 2.0 | 3.5 |
| 1 | 929 | 723 | " | " | " |
| 10 | 215 | 183 | " | " | " |
| 100 | 49.9 | 46.3 | " | " | " |

| | | | | | |
|---|---|---|---|---|---|
| *These samples were not measured at the 0.1 and 1 s⁻¹ shear rates (the shear range was ca 2 - 100 s⁻¹) but the assessment that these exhibit Newtonian flow properties justifies the entries. | | | | | |

The results show that the viscosities of the liquid dosage forms of the invention are dramatically less than that of the commercial formulations of the same drug, even when the drug is present at *more than twice* the concentration as that found in the commercial formulations. In particular, at 90 mg/ml, the liquid dosage forms of the invention have an infinitesimal viscosity of 3.5 mPa·s at a shear rate of 0.1 s⁻¹, as compared to the BMS and PAR liquid dosage forms of 4010 and 2860 mPa·s, respectively.

### Example 2

The purpose of this example was to demonstrate the improved viscosity characteristics of the liquid dosage forms of the invention as compared to conventional liquid dosage forms of the same active agent, naproxen.

Two aqueous nanoparticulate formulations of naproxen were prepared, and the viscosity of these two formulations was then compared to a liquid dosage form of a conventional form (*i.e.,* non-nanoparticulate) of naproxen - NAPROSYN^{®} (Hoffmann-La Roche Inc. (Roche) (Nutley, NJ).

The first nanoparticulate naproxen formulation comprised polyvinylpyrrolidone (PVP) as a surface stabilizer. An aqueous dispersion of 3 wt. % PVP K29/32 and 30 wt. % naproxen was charged into a 2 L recirculation mill (Type: LMZ 2; Mfg.: Netzsh, Inc., Exton, PA). The milling media consisted of PolyMill™ 500 polymeric media (Dow Chemical Co.). The total batch size was 15 kg. The mill was operated at ca 3000 rpm. The batch was harvested after 15 hrs of operation, at which the naproxen particle mean diameter was 105 nm, as measured using a Horiba LA-91 0 particle size analyzer (Irvine, CA).

The second nanoparticulate naproxen formulation comprised lysozyme as a surface stabilizer. An aqueous dispersion of 7.5 wt. % lysozyme and 30 wt. % naproxen was charged into a 2 L recirculation mill (Type: LMZ 2; Mfg.: Netzsh, Inc., Exton, PA). The milling media consisted of PolyMill™ 500 polymeric media (Dow Chemical Co.). The total batch size was 15 kg. The mill was operated at *ca* 3000 rpm. The batch was harvested after 11 hrs of operation, at which the mean particle diameter was 93 nm, as measured using a Horiba LA-910 particle size analyzer (Irvine, CA).

The viscosity of the two dispersions of nanoparticulate naproxen was then compared with the viscosity of NAPROSYN^{®}, as shown in Table 4, below.

| **TABLE 4** | | | |
|---|---|---|---|
| **Shear Rate** | **NAPROSYN^{®}** | **Naproxen/PVP*** | **Naproxen/ Lysozyme** |
| **s⁻¹** | **mPa s** | **mPa s** | **mPa s** |
| 0.1 | 7982 | 17 | 777 |
| 1 | 1095 | 17 | 195 |
| 10 | 150 | 17 | 49 |
| 100 | 21 | 17 | 12 |

| | | | |
|---|---|---|---|
| * Sample not measured at 0.1 and 1 s-1 shear rates (actual range was ca 2 - 100) but the Newtonian flow behavior justifies the entries. | | | |

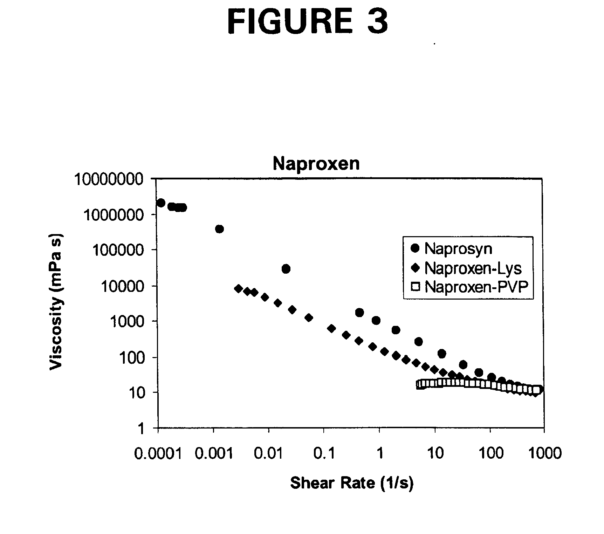

The results shown in Table 4 demonstrate that the liquid nanoparticulate naproxen compositions have a dramatically lower viscosity as compared to the liquid dosage form of a conventional non-nanoparticulate naproxen composition. See also Figure 3, which shows a graphical comparison of the viscosity values given in Table 4.

### Example 3

The purpose of this example was to demonstrate the improved viscosity characteristics of the liquid dosage forms of the invention as compared to conventional liquid dosage forms of the same active agent, Compound A, which is a COX-2 inhibitor.

A nanoparticulate dispersion of COMPOUND A having 25% (w/w) COMPOUND A, 5% copovidone (w/w), and 0.357% docusate sodium (DOSS) (w/w) was milled for 4.5 hours under high energy milling conditions in a DYNO^{®}-Mill KDL (Willy A. Bachofen AG, Maschinenfabrik, Basel, Switzerland) equipped with a 300 cc recirculation chamber and utilizing 500 µm polymeric attrition media (Dow Chemical Co.). Following milling, the final mean particle size of the Compound A particles was 117 nm. Particle size analysis was performed with a Horiba LA-910 particle size analyzer (Irvine, CA).

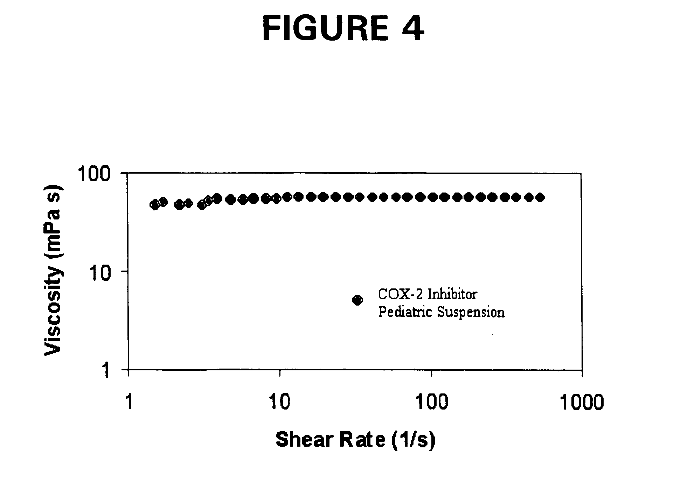

240 g of the milled nanoparticulate Compound A dispersion was then added to a solution containing 20 g sucrose, 0.8 g methyl paraben, 0.04 g propyl paraben, 0.6 g citric acid monohydrate, 1500 g glycerol, and 238 g water. The average viscosity of this composition was 54 mPa s. Figure 4 shows the viscosity of the Compound A dispersion as compared to the shear rate, with the results indicating a consitently uniform, low viscosity.

The dispersion was then divided into four aliquots and varying flavors and colors were added to each aliquot. The composition of each sample is shown below in Tables 5. 6, 7, and 8.

**TABLE 5**

| **Composition of Sample #1 Utilizing the Nanoparticulate** | | |
|---|---|---|
| **Dispersion of Compound A (COX-2 Inhibitor)** | | |
| **Tutti-Fruitti** | **wt%** | **g / kg** |
| Compound A | 3.000% | 30.000 |
| Plasdone^{®} S-630* | 0.600% | 6.000 |
| Docusate Sodium (DOSS) | 0.043% | 0.429 |
| Glycerol | 75.000% | 750.000 |
| Methyl Paraben Sodium | 0.040% | 0.400 |
| Propyl Paraben Sodium | 0.005% | 0.050 |
| Citric Acid Monohydrate | 0.030% | 0.300 |
| Sucrose | 1.000% | 10.000 |
| Tutti-Fruitti Flavor (Firmenich 501040 A) | 0.010% | 0.100 |
| Red # 40 | 0.004% | 0.040 |
| Blue # 1 | 0.000% | 0.000 |
| SWFI | 20.271% | 202.681 |
| | **TOTAL** | 1,000 |

| | | |
|---|---|---|
| * random copolymers of vinyl acetate and vinyl pyrrolidone | | |

**TABLE 6**

| **Composition of Sample #2 Utilizing the Nanoparticulate** | | |
|---|---|---|
| **Dispersion of Compound A (COX-2 Inhibitor)** | | |
| **Cherry** | **wt%** | **g/kg** |
| Compound A | 3.000% | 30.000 |
| Plasdone^{®} S-630 | 0.600% | 6.000 |
| Docusate Sodium | 0.043% | 0.429 |
| Glycerol | 75.000% | 750.000 |
| Methyl Paraben Sodium | 0.040% | 0.400 |
| Propyl Paraben Sodium | 0.005% | 0.050 |
| Citric Acid Monohydrate | 0.030% | 0.300 |
| Sucrose | 1.000% | 10.000 |
| Art Cherry Flavor (Firmenich 501467 A) | 0.010% | 0.100 |
| Red # 40 | 0.004% | 0.040 |
| Blue # 1 | 0.000% | 0.000 |
| SWFI | 20.271% | 202.681 |
| | **TOTAL** | 1,000 |

**TABLE 7**

| **Composition of Sample #3 Utilizing the Nanoparticulate** | | |
|---|---|---|
| **Dispersion of Compound A (COX-2 inhibitor)** | | |
| **Grape** | **wt%** | **g/kg** |
| Compound A | 3.000% | 30.000 |
| Plasdone^{®} S-630 | 0.600% | 6.000 |
| Docusate Sodium | 0.043% | 0.429 |
| Glycerol | 75.000% | 750.000 |
| Methyl Paraben Sodium | 0.040% | 0.400 |
| Propyl Paraben Sodium | 0.005% | 0.050 |
| Citric Acid Monohydrate | 0.030% | 0.300 |
| Sucrose | 1.000% | 10.000 |
| Grape Flavor (Firmenich 501040 A) | 0.050% | 0.100 |
| Red # 40 | 0.001% | 0.010 |
| Blue # 1 | 0.002% | 0.020 |
| SWFI | 20.271 % | 202.691 |
| | **TOTAL** | 1,000 |

**TABLE 8**

| **Composition of Sample #4 Utilizing the Nanoparticulate** | | |
|---|---|---|
| **Dispersion of Compound A (COX-2 Inhibitor)** | | |
| **Bubble Gum** | **wt%** | **g /kg** |
| Compound A | 3.000% | 30.000 |
| Plasdone^{®} S-630 | 0.600% | 6.000 |
| Docusate Sodium | 0.043% | 0.429 |
| Glycerol | 75.000% | 750.000 |
| Methyl Paraben Sodium | 0.040% | 0.400 |
| Propyl Paraben Sodium | 0.005% | 0.050 |
| Citric Acid Monohydrate | 0.030% | 0.300 |
| Sucrose | 1.000% | 10.000 |
| Bubble Gum Flavor (Flavors of NA # 815.065 / WN) | 0.150% | 0.150 |
| Red # 40 | 0.004% | 0.040 |
| Blue # 1 | 0.000% | 0.000 |
| SWFI | 20.271% | 202.631 |
| | **TOTAL** | 1,000 |

It will be apparent to those skilled in the art that various modifications and variations can be made in the methods and compositions of the present invention without departing from the spirit or scope of the invention. Thus, it is intended that the present invention cover the modifications and variations of this invention provided they come within the scope of the appended claims and their equivalents.
The following pages 49 to 79 contain prefered embodiments.
Accordingly, the term "claim" as used therein refers to such a "prefered embodiment".
1. A low viscosity liquid dosage form comprising:
   (a) particles of at least one active agent;
   (b) at least one surface stabilizer; and
   (c) at least one pharmaceutically acceptable excipient, carrier, or a combination thereof,
   wherein: (i) the active agent particles have an effective average particle size of less than about 2 microns and (ii) the dosage form has a viscosity of less than about 2000 mPa·s at a shear rate of 0.1 (1/s).
2. The dosage form of claim 1 having a viscosity at a shear rate of 0.1 (1/s) selected from the group consisting of from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 1 25 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to about 1 mPa·s.
3. The dosage form of claim 1 or claim 2, wherein the viscosity of the dosage form is selected from the group consisting of less than about 1/200, less than about 1/100, less than about 1/50, less than about 1/25, and less than about 1 /10 of the viscosity of a standard conventional liquid dosage form of the same active agent at about the same concentration per ml of active agent.
4. The dosage form of any one of claims 1-3, wherein the viscosity of the dosage form is selected from the group consisting of less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, and less than about 90% of the viscosity of a standard conventional liquid dosage form of the same active agent at about the same concentration per ml of active agent.
5. The dosage form of any one of claims 1-4, wherein the amount of the active agent per ml is equal to or greater than the amount of the active agent per ml of a standard conventional liquid dosage form of the same active agent.
6. The dosage form of any one of claims 1-5 formulated for administration selected from the group consisting of oral, pulmonary, rectal, ophthalmic, colonic, parenteral, intracisternal, intravenous, intravaginal, intraperitoneal, local, buccal, nasal, and topical administration.
7. The dosage form of any one of claims 1-6 suitable for administration in a form selected from the group consisting of controlled release administration, fast melt administration, and aerosol administration.
8. The dosage form of any one of claims 1-7, wherein the effective average particle size of the active agent is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
9. The dosage form of any one of claims 1-8, wherein at least about 70%, about 90%, or about 95% of the active agent particles have a particle size less than the effective average particle size.
10. The dosage form of any one of claims 1-9, wherein said active agent is water-soluble.
11. The dosage form of any one of claims 1-9, wherein said active agent is poorly water-soluble.
12. The dosage form of any one of claims 1-11, wherein the active agent is in the form of crystalline particles, semi-crystalline particles, amorphous particles, semi-amorphous particles, or a mixture thereof.
13. The dosage form of any one of claims 1-12, wherein the active agent is present in an amount selected from the group consisting of from about 99.5% to about 0.001 %, from about 95% to about 0.1 %, and from about 90% to about 0.5%, by weight, based on the total combined weight of the at least one active agent and at least one surface stabilizer, not including other excipients.
14. The dosage form of any one of claims 1-13, wherein the at least one active agent is selected from the group consisting of COX-2 inhibitors, anticancer agents, NSAIDS, proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.
15. The dosage form of claim 14, wherein the nutraceutical is selected from the group consisting of dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.
16. The dosage form of any one of claims 1-15, comprising at least two surface stabilizers.
17. The dosage form of any one of claims 1-16, wherein the at least one surface stabilizer is present in an amount selected from the group consisting of from about 0.5% to about 99.999%, from about 5.0% to about 99.9%, and from about 10% to about 99.5%, by weight, based on the total combined dry weight of the at least one active agent and at least one surface stabilizer, not including other excipients.
18. The dosage form of any one of claims 1-17, wherein the at least one surface stabilizer is selected from the group consisting of an anionic surface stabilizer, a cationic surface stabilizer, an ionic surface stabilizer, and a zwitterionic surface stabilizer.
19. The dosage form of claim 18, wherein the at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-derivatized phospholipid, PEG-derivatized cholesterol, PEG-derivatized cholesterol derivative, PEG-derivatized vitamin A, PEG-derivatized vitamin E, and random copolymers of vinyl acetate and vinyl pyrrolidone.
20. The dosage form of claim 18, wherein the at least one cationic surface stabilizer is selected from the group consisting of a polymer, a biopolymer, a polysaccharide, a cellulosic, an alginate, a nonpolymeric compound, a phospholipid, zwitterionic stabilizers, poly-n-methylpyridinium, anthryul pyridinium chloride, chitosan, polylysine, polyvinylimidazole, polybrene, polymethylmethacrylate trimethylammoniumbromide bromide (PMMTMABr), hexyldesyltrimethylammonium bromide (HDMAB), polyvinylpyrrolidone-2-dimethylaminoethyl methacrylate dimethyl sulfate, 1,2 Dipalmitoyl-sn-Glycero-3-Phosphoethanolamine-N-[Amino(Polyethylene Glycol)2000] (sodium salt), Poly(2-methacryloxyethyl trimethylammonium bromide), poloxamines, lysozyme, alginic acid, carrageenan, and POLYOX.
21. The dosage form of claim 18, wherein the at least one cationic surface stabilizer is selected from the group consisting of cationic lipids, sulfonium, phosphonium, quarternary ammonium compounds, stearyltrimethylammonium chloride, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, C₁₂₋₁₅dimethyl hydroxyethyl ammonium chloride, C₁₂₋₁₅dimethyl hydroxyethyl ammonium bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts, amines, protonated quaternary acrylamides, methylated quaternary polymers, and cationic guar.
22. The dosage form of claim 21, wherein the amine is selected from the group consisting of alkylamines, dialkylamines, alkanolamines, polyethylenepolyamines, N,N-dialkylaminoalkyl acrylates, vinyl pyridine, amine salts, lauryl amine acetate, stearyl amine acetate, alkylpyridinium salt, alkylimidazolium salt, amine oxides, and, imide azolinium salts.
23. The dosage form of claim 20, wherein the cationic surface stabilizer is a non polymeric compound selected from the group consisting of benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCI, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.
24. The dosage form according to any of claims 18, 20, 21, 22, or 23, wherein the dosage form is bioadhesive.
25. A method of improving a conventional solid dosage form of an active agent comprising:
   (a) identifying a conventional solid dosage form of an active agent having at least one undesirable trait, wherein the undesirable trait is selected from the group consisting of poor dose uniformity, low dose loading, large size, poor bioavailability, slow onset of activity; poor active agent retention in blood and tumors, significant fed-fasted variability; and
   (b) formulating the active agent into a low viscosity liquid dosage form comprising:
      (i) particles of at least one active agent;
      (ii) at least one surface stabilizer; and
      (iii) at least one pharmaceutically acceptable excipient, carrier, or a combination thereof,
   wherein: (1) the active agent particles have an effective average particle size of less than about 2 microns and (2) the dosage form has a viscosity of less than about 2000 mPa·s at a shear rate of 0.1 (1/s).
26. A method of improving a conventional liquid dosage form of an active agent comprising:
   (a) identifying a conventional solid dosage form of an active agent having at least one undesirable trait, wherein the undesirable trait is selected from the group consisting of high viscosity, poor taste, grittiness, poor bioavailability, slow onset of activity, presence of thickening agents, poor dose loading, poor performance characteristics for oral, intravenous, subcutaneous, or intramuscular injection, presence of organic solvents, presence of a pH extreme, poor active agent retention in blood and tumors, significant fed-fasted variability, high dose volume, poor suitability for parenteral administration, and an inability to be sterile filtered; and
   (b) formulating the active agent into a low viscosity liquid dosage form comprising:
      (i) particles of at least one active agent;
      (ii) at least one surface stabilizer; and
      (iii) at least one pharmaceutically acceptable excipient, carrier, or a combination thereof,
   wherein: (1) the active agent particles have an effective average particle size of less than about 2 microns and (2) the dosage form has a viscosity of less than about 2000 mPa·s at a shear rate of 0.1 (1/s).
27. A method of making a liquid dosage form comprising contacting particles of at least one active agent with at least one surface stabilizer and at least one pharmaceutically acceptable excipient, carrier, or a combination thereof for a time and under conditions sufficient to provide a nanoparticulate composition of the active agent, wherein:
   (a) the active agent particles have an effective average particle size of less than about 2 microns; and
   (b) the liquid dosage form has a viscosity of less than about 2000 mPa·s at a shear rate of 0.1 (1/s).
28. The method of claim 27, wherein said contacting comprising grinding.
29. The method of claim 28, wherein said grinding comprising wet grinding.
30. The method of claim 27, wherein said contacting comprises homogenizing.
31. The method of claim 27, wherein said contacting comprises:
   (a) dissolving the particles of at least one active agent in a solvent;
   (b) adding the resulting solution of the active agent to a solution comprising at least one surface stabilizer; and
   (c) precipitating the solubilized active agent and at least one surface stabilizer by the addition thereto of a non-solvent.
32. The method of any one of claims 27-31, wherein the viscosity of the dosage form is selected from the group consisting of from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to about 1 mPa·s.
33. The method of any one of claims 27-32, wherein the viscosity of the dosage form is selected from the group consisting of less than about 1/200, less than about 1/100, less than about 1 /50, less than about 1/25, and less than about 1/10 of the viscosity of a standard conventional liquid dosage form of the same active agent at about the same concentration per ml of active agent.
34. The method of any one of claims 27-33, wherein the viscosity of the dosage form is selected from the group consisting of less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, and less than about 90% of the viscosity of a standard conventional liquid dosage form of the same active agent at about the same concentration per ml of active agent.
35. The method of any one of claims 27-34, wherein the amount of active agent per ml is equal to or greater than the amount of the same active agent per ml of a standard conventional liquid dosage form of the active agent.
36. The method of any one of claims 27-35, wherein the dosage form is formulated for administration selected from the group consisting of oral, pulmonary, rectal, ophthalmic, colonic, parenteral, intracisternal, intravenous, intravaginal, intraperitoneal, local, buccal, nasal, and topical administration.
37. The method of any one of claims 27-36, wherein the dosage form is suitable for administration in a form selected from the group consisting of controlled release administration, fast melt administration, and aerosol administration.
38. The method of any one of claims 27-37, wherein the effective average particle size of the active agent is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
39. The method of any one of claims 27-38, wherein at least about 70%, about 90%, or about 95% of the active agent particles have a particle size less than the effective average particle size.
40. The method of any one of claims 27-39, wherein the active agent is water-soluble.
41. The method of any one of claims 27-39, wherein the active agent is poorly water-soluble.
42. The method of any one of claims 27-41, wherein the active agent is in the form of crystalline particles, semi-crystalline particles, amorphous particles, semi-amorphous particles, or a mixture thereof.
43. The method of any one of claims 27-42, wherein the active agent is present in an amount selected from the group consisting of from about 99.5% to about 0.001 %, from about 95% to about 0.1 %, and from about 90% to about 0.5%, by weight, based on the total combined weight of the at least one active agent and at least one surface stabilizer, not including other excipients.
44. The method of any one of claims 27-43, wherein the at least one active agent is selected from the group consisting of COX-2 inhibitors, anticancer agents, NSAIDS, proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.
45. The method of claim 44, wherein the nutraceutical is selected from the group consisting of dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.
46. The method of any one of claims 27-45, comprising at least two surface stabilizers.
47. The method of any one of claims 27-46, wherein the at least one surface stabilizer is present in an amount selected from the group consisting of from about 0.5% to about 99.999%, from about 5.0% to about 99.9%, and from about 10% to about 99.5%, by weight, based on the total combined dry weight of the at least one active agent and at least one surface stabilizer, not including other excipients.
48. The method of any one of claims 27-47, wherein the at least one surface stabilizer is selected from the group consisting of an anionic surface stabilizer, a cationic surface stabilizer, an ionic surface stabilizer, and a zwitterionic surface stabilizer.
49. The method of claim 48, wherein the at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-derivatized phospholipid, PEG-derivatized cholesterol, PEG-derivatized cholesterol derivative, PEG-derivatized vitamin A, PEG-derivatized vitamin E, and random copolymers of vinyl acetate and vinyl pyrrolidone.
50. The method of claim 48, wherein the at least one cationic surface stabilizer is selected from the group consisting of a polymer, a biopolymer, a polysaccharide, a cellulosic, an alginate, a nonpolymeric compound, a phospholipid, zwitterionic stabilizers, poly-n-methylpyridinium, anthryul pyridinium chloride, chitosan, polylysine, polyvinylimidazole, polybrene, polymethylmethacrylate trimethylammoniumbromide bromide (PMMTMABr), hexyldesyltrimethylammonium bromide (HDMAB), polyvinylpyrrolidone-2-dimethylaminoethyl methacrylate dimethyl sulfate, 1,2 Dipalmitoyl-sn-Glycero-3-Phosphoethanolamine-N-[Amino(Polyethylene Glycol)2000] (sodium salt), Poly(2-methacryloxyethyl trimethylammonium bromide), poloxamines, lysozyme, alginic acid, carrageenan, and POLYOX.
51. The method of claim 48, wherein the at least one cationic surface stabilizer is selected from the group consisting of cationic lipids, sulfonium, phosphonium, quarternary ammonium compounds, stearyltrimethylammonium chloride, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, C₁₂₋₁₅dimethyl hydroxyethyl ammonium chloride, C₁₂₋₁₅dimethyl hydroxyethyl ammonium bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts, amines, protonated quaternary acrylamides, methylated quaternary polymers, and cationic guar.
52. The method of claim 51, wherein the amine is selected from the group consisting of alkylamines, dialkylamines, alkanolamines, polyethylenepolyamines, N,N-dialkylaminoalkyl acrylates, vinyl pyridine, amine salts, lauryl amine acetate, stearyl amine acetate, alkylpyridinium salt, alkylimidazolium salt, amine oxides, and, imide azolinium salts.
53. The method of claim 50, wherein the cationic surface stabilizer is a nonpolymeric compound selected from the group consisting of benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-1 8 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCI, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.
54. The method of according to any of claims 48, 50, 51, 52, or 53, wherein the dosage form is bioadhesive.
55. A method of treating a subject in need with a liquid dosage form of a nanoparticulate active agent comprising administering to the subject an effective amount of a liquid dosage form comprising:
   (a) particles of at least one active agent;
   (b) at least one surface stabilizer; and
   (c) at least one pharmaceutically acceptable excipient, carrier, or a combination thereof,
   wherein: (i) the active agent particles have an effective average particle size of less than about 2 microns and (ii) the liquid dosage form has a viscosity of less than about 2000 mPa·s at a shear rate of 0.1 (1/s).
56. The method of claim 55, wherein the dosage form has a viscosity selected from the group consisting of from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to about 1 mPa·s.
57. The method of claim 55 or claim 56, wherein the viscosity of the dosage form is selected from the group consisting of less than about 1/200, less than about 1/100, less than about 1/50, less than about 1/25, and less than about 1 /10 of the viscosity of a standard conventional liquid oral formulation of the same active agent at about the same concentration per ml of active agent.
58. The method of any one of claims 55-57, wherein the viscosity of the dosage form is selected from the group consisting of less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, and less than about 90% of the viscosity of a standard conventional liquid dosage form of the same active agent at about the same concentration per ml of active agent.
59. The method of any one of claims 55-58, wherein the amount of the active agent per ml is equal to or greater than the amount of the active agent per ml of a standard conventional liquid formulation of the same active agent.
60. The method of any one of claims 55-59, wherein the dosage form is formulated for administration selected from the group consisting of oral, pulmonary, rectal, ophthalmic, colonic, parenteral, intracisternal, intravenous, intravaginal, intraperitoneal, local, buccal, nasal, and topical administration.
61. The method of any one of claims 55-60, wherein the dosage form is suitable for administration in a form selected from the group consisting of controlled release administration, fast melt administration, and aerosol administration.
62. The method of any one of claims 55-61, wherein the effective average particle size of the active agent is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
63. The method of any one of claims 55-62, wherein at least about 70%, about 90%, or about 95% of the active agent particles have a particle size less than the effective average particle size.
64. The method of any one of claims 55-63, wherein the active agent is water-soluble.
65. The method of any one of claims 55-63, wherein the active agent is poorly water-soluble.
66. The method of any one of claims 55-65, wherein the active agent is in the form of crystalline particles, semi-crystalline particles, amorphous particles, semi-amorphous particles, or a mixture thereof.
67. The method of any one of claims 55-66, wherein the active agent is present in an amount selected from the group consisting of from about 99.5% to about 0.001 %, from about 95% to about 0.1 %, and from about 90% to about 0.5%, by weight, based on the total combined weight of the at least one active agent and at least one surface stabilizer, not including other excipients.
68. The method of any one of claims 55-67, wherein the at least one active agent is selected from the group consisting of COX-2 inhibitors, anticancer agents, NSAIDS, proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.
69. The method of claim 68, wherein the nutraceutical is selected from the group consisting of dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.
70. The method of any one of claims 55-69, comprising at least two surface stabilizers.
71. The method of any one of claims 55-70, wherein the at least one surface stabilizer is present in an amount selected from the group consisting of from about 0.5% to about 99.999%, from about 5.0% to about 99.9%, and from about 10% to about 99.5%, by weight, based on the total combined dry weight of the at least one active agent and at least one surface stabilizer, not including other excipients.
72. The method of any one of claims 55-71, wherein the at least one surface stabilizer is selected from the group consisting of an anionic surface stabilizer, a cationic surface stabilizer, an ionic surface stabilizer, and a zwitterionic surface stabilizer.
73. The method of claim 72, wherein the at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-mattopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-derivatized phospholipid, PEG-derivatized cholesterol, PEG-derivatized cholesterol derivative, PEG-derivatized vitamin A, PEG-derivatized vitamin E, and random copolymers of vinyl acetate and vinyl pyrrolidone.
74. The method of claim 72, wherein the at least one cationic surface stabilizer is selected from the group consisting of a polymer, a biopolymer, a polysaccharide, a cellulosic, an alginate, a nonpolymeric compound, a phospholipid, zwitterionic stabilizers, poly-n-methylpyridinium, anthryul pyridinium chloride, chitosan, polylysine, polyvinylimidazole, polybrene, polymethylmethacrylate trimethylammoniumbromide bromide (PMMTMABr), hexyldesyltrimethylammonium bromide (HDMAB), polyvinylpyrrolidone-2-dimethylaminoethyl methacrylate dimethyl sulfate, 1,2 Dipalmitoyl-sn-Glycero-3-Phosphoethanolamine-N-[Amino(Polyethylene Glycol)2000] (sodium salt), Poly(2-methacryloxyethyl trimethylammonium bromide), poloxamines, lysozyme, alginic acid, carrageenan, and POLYOX.
75. The method of claim 72, wherein the at least one cationic surface stabilizer is selected from the group consisting of cationic lipids, sulfonium, phosphonium, quarternary ammonium compounds, stearyltrimethylammonium chloride, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, C₁₂₋₁₅dimethyl hydroxyethyl ammonium chloride, C₁₂₋₁₅dimethyl hydroxyethyl ammonium bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyidimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts, amines, protonated quaternary acrylamides, methylated quaternary polymers, and cationic guar.
76. The method of claim 75, wherein the amine is selected from the group consisting of alkylamines, dialkylamines, alkanolamines, polyethylenepolyamines, N,N-dialkylaminoalkyl acrylates, vinyl pyridine, amine salts, lauryl amine acetate, stearyl amine acetate, alkylpyridinium salt, alkylimidazolium salt, amine oxides, and, imide azolinium salts.
77. The method of claim 72, wherein the cationic surface stabilizer is a nonpolymeric compound selected from the group consisting of benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCI, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.
78. The method according to any of claims 72, 74, 75, 76, or 77, wherein the dosage form is bioadhesive.
79. The method of any one of claims 55-78, wherein the condition to be treated is selected from the group consisting of neoplastic diseases, breast cancer, endometrial cancer, uterine cancer, cervical cancer, prostate cancer, renal cancer, hormone replacement therapy in post-menopausal women, endometriosis, hirsutism, dysmenorrhea, uterine bleeding, HIV wasting, cancer wasting, migraine headache, cachexia, anorexia, castration, oral contraception, motion sickness, emesis related to cytotoxic drugs, gastritis, ulcers, dyspepsia, gastroenteritis, including collitis and food poisoning, inflammatory bowel disease, Crohn's disease, migraine headaches, and any other condition which is accompanied by the symptoms of nausea and vomiting.
80. The method of any one of claims 55-79, wherein the condition to be treated is selected from the group consisting of pain, inflammation, arthritis, cancer, kidney disease, osteoporosis, Alzheimer's disease, and familial adenomatous polyposis.
81. The method of claim 80, wherein the condition to be treated is selected from the group consisting of osteoarthritis, rheumatoid arthritis, juvenile arthritis, gout, ankylosing spondylitis, systemic lupus erythematosus, bursitis, tendinitis, myofascial pain, carpal tunnel syndrome, fibromyalgia syndrome, infectious arthritis, psoriatic arthritis, reiter's syndrome, and scleroderma.
82. The method of claim 79, 80, or 81, wherein said subject is a human.

## Claims

1. A low viscosity liquid dosage form comprising:
(a) particles of at least one active agent having an effective average particle size of less than about 2 microns;
(b) at least one surface stabilizer; and
(c) at least one pharmaceutically acceptable excipient, carrier, or a combination thereof,
wherein the dosage form has a viscosity of less than about 2000 mPa·s at a shear rate of 0.1 (1/s).

2. The dosage form of claim 1:
(a) having a viscosity at a shear rate of 0.1 (1/s) selected from the group consisting of from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to about 1 mPa·s; and/or
(b) wherein the viscosity of the dosage form is selected from the group consisting of less than about 1/200, less than about 1/100, less than about 1/50, less than about 1/25, and less than about 1/10 of the viscosity of a standard conventional liquid dosage form of the same active agent at about the same concentration per ml of active agent; and/or
(c) wherein the viscosity of the dosage form is selected from the group consisting of less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, and less than about 90% of the viscosity of a standard conventional liquid dosage form of the same active agent at about the same concentration per ml of active agent.

3. The dosage form of claim 1 or claim 2, wherein the amount of the active agent per ml is equal to or greater than the amount of the active agent per ml of a standard conventional liquid dosage form of the same active agent.

4. The dosage form of any one of claims 1 to 3:
(a) formulated for administration selected from the group consisting of oral, pulmonary, rectal, ophthalmic, colonic, parenteral, intracisternal, intravenous, intravaginal, intraperitoneal, local, buccal, nasal, and topical administration; and/or
(b) suitable for administration in a form selected from the group consisting of controlled release administration, fast melt administration, and aerosol administration.

5. The dosage form of any one of claims 1 to 4, wherein the effective average particle size of the active agent is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

6. The dosage form of any one of claims 1 to 5, wherein at least about 70%, about 90%, or about 95% of the active agent particles have a particle size less than the effective average particle size.

7. The dosage form of any one of claims 1 to 6, wherein said active agent is water-soluble or poorly water-soluble.

8. The dosage form of any one of claims 1 to 7, wherein the active agent is in the form of crystalline particles, semi-crystalline particles, amorphous particles, semi-amorphous particles, or a mixture thereof.

9. The dosage form of any one of claims 1 to 8, wherein:
(a) the active agent is present in an amount selected from the group consisting of from about 99.5% to about 0.001%, from about 95% to about 0.1%, and from about 90% to about 0.5%, by weight, based on the total combined weight of the at least one active agent and at least one surface stabilizer, not including other excipients; and/or
(b) the at least one surface stabilizer is present in an amount selected from the group consisting of from about 0.5% to about 99.999%, from about 5.0% to about 99.9%, and from about 10% to about 99.5%, by weight, based on the total combined dry weight of the at least one active agent and at least one surface stabilizer, not including other excipients.

10. The dosage form of any one of claims 1 to 9, wherein the at least one active agent is selected from the group consisting of COX-2 inhibitors, anticancer agents, NSAIDS, proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.

11. The dosage form of claim 10, wherein the nutraceutical is selected from the group consisting of dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.

12. The dosage form of any one of claims 1 to 11, comprising at least two surface stabilizers.

13. The dosage form of any one of claims 1 to 12, wherein the at least one surface stabilizer is selected from the group consisting of an anionic surface stabilizer, a cationic surface stabilizer, an ionic surface stabilizer, and a zwitterionic surface stabilizer.

14. The dosage form of any one of claims 1 to 13, wherein the at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-derivatized phospholipid, PEG-derivatized cholesterol, PEG-derivatized cholesterol derivative, PEG-derivatized vitamin A, PEG-derivatized vitamin E, random copolymers of vinyl acetate and vinyl pyrrolidone, cationic polymers, cationic biopolymers, cationic polysaccharides, cationic cellulosics, cationic alginate, cationic nonpolymeric compounds, cationic phospholipids, zwitterionic stabilizers, poly-n-methylpyridinium, anthryul pyridinium chloride, chitosan, polylysine, polyvinylimidazole, polybrene, polymethylmethacrylate trimethylammoniumbromide bromide (PMMTMABr), hexyldesyltrimethylammonium bromide (HDMAB), polyvinylpyrrolidone-2-dimethylaminoethyl methacrylate dimethyl sulfate, 1,2 Dipalmitoyl-sn-Glycero-3-Phosphoethanolamine-N-[Amino(Polyethylene Glycol)2000] (sodium salt), Poly(2-methacryloxyethyl trimethylammonium bromide), poloxamines, lysozyme, alginic acid, carrageenan, POLYOX, cationic lipids, sulfonium, phosphonium, quarternary ammonium compounds, stearyltrimethylammonium chloride, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, C₁₂₋₁₅dimethyl hydroxyethyl ammonium chloride, C₁₂₋₁₅-dimethyl hydroxyethyl ammonium bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈-)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts, amines, protonated quaternary acrylamides, methylated quaternary polymers, cationic guar, benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCI, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

15. The dosage form of claim 14, wherein the amine is selected from the group consisting of alkylamines, dialkylamines, alkanolamines, polyethylenepolyamines, N,N-dialkylaminoalkyl acrylates, vinyl pyridine, amine salts, lauryl amine acetate, stearyl amine acetate, alkylpyridinium salt, alkylimidazolium salt, amine oxides, and, imide azolinium salts.

16. The dosage form of any one of claims 1 to 15, wherein the dosage form is bioadhesive.

17. Use of the dosage form of any one of claims 1 to 16 for the manufacture of a medicament.

18. The use of claim 17, wherein the medicament is useful in treating a condition selected from the group consisting of neoplastic diseases, breast cancer, endometrial cancer, uterine cancer, cervical cancer, prostate cancer, renal cancer, hormone replacement therapy in post-menopausal women, endometriosis, hirsutism, dysmenorrhea, uterine bleeding, HIV wasting, cancer wasting, migraine headache, cachexia, anorexia, castration, oral contraception, motion sickness, emesis related to cytotoxic drugs, gastritis, ulcers, dyspepsia, gastroenteritis, including collitis and food poisoning, inflammatory bowel disease, Crohn's disease, migraine headaches, any condition which is accompanied by the symptoms of nausea and vomiting, pain, inflammation, arthritis, cancer, kidney disease, osteoporosis, Alzheimer's disease, and familial adenomatous polyposis, osteoarthritis, rheumatoid arthritis, juvenile arthritis, gout, ankylosing spondylitis, systemic lupus erythematosus, bursitis, tendinitis, myofascial pain, carpal tunnel syndrome, fibromyalgia syndrome, infectious arthritis, psoriatic arthritis, reiter's syndrome, and scleroderma.

19. A method of improving a conventional solid or liquid dosage form of an active agent comprising:
(a) identifying a conventional solid or liquid dosage form of an active agent having at least one undesirable trait, wherein the undesirable trait is selected from the group consisting of poor dose uniformity, low or poor dose loading, large size, poor bioavailability, slow onset of activity, poor active agent retention in blood and tumors, significant fed-fasted variability, high viscosity, poor taste, grittiness, presence of thickening agents, poor performance characteristics for oral, intravenous, subcutaneous, or intramuscular injection, presence of organic solvents, presence of a pH extreme, high dose volume, poor suitability for parenteral administration, and an inability to be sterile filtered; and
(b) formulating the active agent into a low viscosity liquid dosage form comprising:
(i) particles of at least one active agent having an effective average particle size of less than about 2 microns;
(ii) at least one surface stabilizer; and
(iii) at least one pharmaceutically acceptable excipient, carrier, or a combination thereof,
wherein the dosage form has a viscosity of less than about 2000 mPa·s at a shear rate of 0.1 (1/s).

20. A method of making a liquid dosage form comprising contacting particles of at least one active agent with at least one surface stabilizer and at least one pharmaceutically acceptable excipient, carrier, or a combination thereof for a time and under conditions sufficient to provide a nanoparticulate composition of the active agent, wherein:
(a) the active agent particles have an effective average particle size of less than about 2 microns; and
(b) the liquid dosage form has a viscosity of less than about 2000 mPa·s at a shear rate of 0.1 (1/s).
| **New Claim** | **Corresponding PCT Claim(s)** |
|---|---|
| 1 | 1 |
| 2 | 2,3,4 |
| 3 | 5 |
| 4 | 6, 7 |
| 5 | 8 |
| 6 | 9 |
| 7 | 10, 11 |
| 8 | 12 |
| 9 | 13, 17 |
| 10 | 14 |
| 11 | 15 |
| 12 | 16 |
| 13 | 18 |
| 14 | 19, 20, 21, 23 |
| 15 | 22 |
| 16 | 24 |
| 17 | 55 |
| 18 | 79, 80, 81 |
| 19 | 25,26 |
| 20 | 27 |
